Europäisches Patentamt

European Patent Office

Office européen des brevets

(11) Publication number: **0 185 824**
**A1**

(12) **EUROPEAN PATENT APPLICATION**

(21) Application number: **84309137.2**

(88) Date of filing: **28.12.84**

(51) Int. Cl.⁴: **C 12 P 13/06**
**C 12 N 9/96**

(43) Date of publication of application:
**02.07.86 Bulletin 86/27**

(84) Designated Contracting States:
**AT BE CH DE FR GB IT LI LU NL SE**

(71) Applicant: **GENEX CORPORATION**
**6110 Executive Boulevard**
**Rockville Maryland 20852(US)**

(72) Inventor: **Hsiao, Hung-Yu**
**15937 Indiana Hills Terrace**
**Derwood Maryland 20855(US)**

(72) Inventor: **Wei, Teng T.**
**3 Sussex Ct.**
**Rockville Maryland 20854(US)**

(74) Representative: **Holmes, Michael John et al,**
**Frank B. Dehn & Co. European Patent Attorneys Imperial**
**House 15-19 Kingsway**
**London, WC2B 6UZ,(GB)**

(54) Stabilization of tetrahydrofolic acid and serine hydroxymethyltransferase in reaction mixtures for the enzymatic cynthesis of L-serine.

(57) A method of stabilizing tetrahydrofolic acid and serine hydroxymethyltransferase in reaction mixtures for the enzymatic synthesis of L-serine by addition to the reaction mixture of a reducing agent or a mixture of reducing agents and by the addition of a high concentration of THF relative to a catalytic amount is disclosed.

EP 0 185 824 A1

Croydon Printing Company Ltd

# STABILIZATION OF TETRAHYDROFOLIC ACID AND SERINE HYDROXYMETHYLTRANSFERASE IN REACTION MIXTURES FOR THE ENZYMATIC SYNTHESIS OF L-SERINE

## Background of the Invention

### Field of the Invention:

The present invention relates to improving the efficiency of enzymatic synthesis of L-serine.

### Description of the Background Art:

The amino acid L-serine is a valuable commercial product that is employed in hyperalimentation and nutritional compositions and is also used as an intermediate or starting material in certain synthetic processes. There is thus a demand for L-serine, and various processes have been developed for producing it, including fermentation, chemical synthesis and enzymatic synthesis.

One method of enzymatic synthesis of L-serine involves reacting glycine and formaldehyde in the presence of the enzyme serine hydroxymethyltransferase and the co-factor tetrahydrofolic acid (see copending U.S. Patent Application Serial No. 442,962). While this method has proven efficient from a production standpoint relative to certain other prior processes, there are special problems that must be addressed while using this process. Tetrahydrofolate (sometimes referred to as THF) is very sensitive to oxidation by oxygen in air and serine hydroxymethyl transferase (sometimes referred to as SHMT) is sensitive to inactivation by formaldehyde.

It is an object of this invention to provide a method of stabilizing THF and SHMT under reaction conditions for the enzymatic synthesis of L-serine, and to thereby improve the efficiency of the process.

2

0185824

## Summary of the Invention

In accordance with the present invention, there is provided a method of stabilizing tetrahydrofolic acid and serine hydroxymethyltransferase in a reaction mixture for the enzymatic synthesis of serine which comprises adding to the reaction mixture a stabilizing amount of a reducing agent or mixture of reducing agents, and a high concentration of THF relative to a catalytic amount.

## Detailed Description of the Invention

The present invention relates to a method of stabilizing tetrahydrofolic acid and serine hydroxymethyltransferase in a reaction mixture for the enzymatic synthesis of L-serine by the addition to the reaction mixture a reducing agent or mixture of reducing agents, and appropriate amounts of THF.

Enzymatic synthesis of L-serine from glycine and formaldehyde generally requires the presence of the enzyme serine hydroxymethyltransferase and the cofactor tetrahydrofolic acid. Formaldehyde reacts with THF to form methylene-THF. Glycine then reacts with methylene-THF in the presence of SHMT to form L-serine and regenerate THF. The synthesis advantageously takes place in a reaction vessel, for example, a stirred tank reactor, with the reaction mixture at a temperature of from about 4°C to about 60°C and at a pH of from about 4 to about 11. High speed of agitation of the mixture is required to facilitate the dispersion of HCHO.

Serine hydroxymethyltransferse (SHMT) is very sensitive to formaldehyde. Formaldehyde (HCHO) at low concentration can quickly inactivate SHMT enzyme. Since formaldehyde is one of reactants necessary to make serine from glycine, a method to prevent SHMT inactivation by formaldehyde is needed to have a successful process. THF is a cofactor for SHMT in the reaction; therefore, a catalytic amount of THF would normally be enough to carry reaction.  THF is recycled in the reaction, shown in the following scheme.

$$HCHO + THF \longrightarrow methylene\text{-}THF$$

$$\underline{methylene\text{-}THF + gly \xrightarrow{\text{SHMT}} Ser + THF}$$

Net reaction   $HCHO + Gly \longrightarrow$ serine

The process of the present invention takes advantage of the high affinity of THF for HCHO (Keq = $3 \times 10^4$).  In the process of the present invention, THF is used to consume HCHO in order to prevent HCHO from inactivating the SHMT.  Therefore, a THF concentration higher than catalytic amount is employed to prevent enzyme inactivation by HCHO. The THF is added in a concentration range between 0.01 mM to 50 mM with a preferred range between 0.1 mM and 5 mM. It has been discovered that excess THF coupled with a formaldehyde addition rate which is rate limiting for the overall reaction prevents formaldehyde accumulation and thus SHMT inactivation.  Accordingly, formaldehyde is preferably added to the reaction mixture at a rate less than or equal to the rate at which it is consumed in the reaction.  The precise rate of addition will vary with the reaction conditions and amount of THF present.  The rate of addition can easily be adjusted by monitoring the buildup of formaldehyde in the reaction medium.

Tetrahydrofolic acid is very unstable under reaction conditions and in the presence of oxygen.  A preferred method of preventing oxygen contact with the reaction mixture is by the induced presence of a non-reacting gas in the reaction vessel.  Particularly preferred non-reacting gases are nitrogen and argon.  Since a high rate of agitation is required for the reaction, it is extremely difficult to maintain an $O_2$-free environment.  If THF is oxidized by air, the protection of SHMT from HCHO by reaction with THF no longer exists.

4

0185824

The SHMT is generally in the reaction mixture in the form of purified enzyme, crude extract or contained in whole cells of microorganisms which have been transformed with expression vectors containing DNA sequences encoding the amino acid sequence of SHMT, under the control of regulatory sequences which direct the expression of SHMT. The instability of THF and thus SHMT under reaction conditions limits the efficiency of the enzymatic process for L-serine synthesis.

It has been discovered that the presence in the reaction mixture of a reducing agent or a mixture of reducing agents stabilizes tetrahydrofolic acid and thus serine hydroxymethyltransferase under reaction conditions, without inhibiting the enzymatic process. This stabilizing effect on THF and SHMT is enhanced by prevention of air contact with the reaction mixture.

Any reducing agent may be employed which does not deleteriously affect the reaction process. Preferred reducing agents are ascorbic acid, thioglycolate and thioalcohols, including mercaptans, e.g., 1,3-dimercaptopropan-2-ol, 2,3-dimercaptopropanol, 1,2-dimercaptoethane, dithiothreitol, dithioerythritol, and mercaptoethanol, L-thiazolidine-4-carboxylic acid as well as amionethyliso-thiouronium bromide. Particularly preferred reducing compounds are mercaptoethanol, dithiothreitol and thioglycolate. 2-mercaptoethanol is most preferred. The concentration of the reducing agent or agents in the reaction mixture is preferably in the range of from about 0.01M to about 0.2M, most preferably from about 0.02M to about 0.1M.

The reducing agent or agents are added to the reaction mixture at the beginning of the reaction, continuously during the reaction, or at the beginning of the reaction and periodically during the reaction. Preferably the reducing agent or agents are added to the reaction mixture at the beginning of the reaction and periodically thereafter during the reaction.

The reducing agent is added in an amount which stabilizes the THF and the SHMT in the reaction mixture. Generally, an amount of reducing agent sufficient to maintain the redox potential of the reation mixture at between -300mV and -500mV will stablize the THF and the SHMT. The redox potential can be monitored by a redox probe and maintained within the range of about -300mV to about -350mV by selective titration of the reducing agent or agents into the reaction mixture. If desired, this can be done by means of an automated feedback system which will monitor the redox potential and add reducing agent as necessary during the reaction. It is particularly preferred to maintain the redox potential at about -300 mV.

Typically, the process of the invention is carried out in the following manner. Glycine, THF and formaldehyde are charged to a reaction vessel in a suitable reaction solvent, together with an initial portion of reducing agent, such as 2-mercaptoethanol, and mixed under a nitrogen blanket. SHMT is then added, for example in the form of whole transformed cells in which the SHMT has been expressed. Preferably, the cell walls have been ruptured in order to allow contact between the expressed enzyme in the cells and the reactants in the reaction medium. This can be achieved by contacting the transformant cells with a detergent such as cetylpyridinium chloride, Triton X-100, Tween 100, NP40 or solvent such as toluene or acetone.

The redox potential is continuously monitored with a probe and maintained at about -300 mV by the addition of reducing agent until the reaction is complete.

The invention is further illustrated by the following examples, which are not intended to be limiting.

Example 1


Tetrahydrofolate (10 mM) in 0.1 M potassium phosphate buffer (pH 7.5) which was degassed by bubbling $N_2$ through for 10 minutes was mixed with various concentrations of 2-mercaptoethanol. This solution was kept in a serum bottle at room temperature, sealed with a rubber stopper and protected from light by aluminum foil. After 20 hours, tetrahydrofolate was totally degraded in the presence of 10 mM mercaptoethanol. About 40% of the tetrahydrofolate was left when 50 mM 2-mercaptoethanol was used. 2-Mercaptoethanol at 100 mM stabilized tetrahydrofolic acid for 16 hours under unstirred conditions.

Methylene tetrahydrofolate was formed by mixing tetrahydrofolate with two equivalents of HCHO for 15 minutes at room temperature. The stability test on methylene tetrahydrofolic acid which is identical to the one for tetrahydrofolic acid described above indicated that 10 mM 2-mercaptoethanol is enough to stabilize methylene tetrahydrofolic acid for 20 hours.

Example 2


Tetrahydrofolate (10 mM) in 50 ml of 0.1 M potassium phosphate buffer (pH 7.5) which had been degassed by bubbling $N_2$ through for 10 minutes was mixed with 2-mercaptoethanol (100 mM, final concentration) at 37°C. A stream of $N_2$ gas was maintained over the tetrahydrofolate solution which was under continuous stirring. After 20 hours, both of tetrahydrofolate and methylene tetrahydrofolate were stable.

## Example 3

Serine hydroxymethyltransferase (crude extract from E. coli strain Gx1703 containing pGx122, deposited as NRRL No. B-15215) in 0.1 M potassium phosphate buffer (pH 8.0) was mixed with various concentrations of 2-mercaptoethanol at 37°C and under shaking. The enzyme activity was assayed by two methods described below.

(1)     -phenylserine assay An appropriate amount of enzyme was mixed with 30 mM  -phenylserine solution in 0.1 M potassium-phosphate buffer (pH 8.0) containing 50  m pyridoxal-5'-phosphate at 30°C. A reading of absorbance at 279 was used to indicate enzyme activity.

(2)     Glycine-Serine assay An appropriate amount of enzyme was mixed with 10 mM glycine, HCHO and tetrahydrofolate in 0.1 M potassium phosphate at 8.0. Serine produced at different time intervals was determined by HPLC.

Results of both assays indicated that serine hydroxymethyltransferase was stable in the presence of 0.05 M, 0.1 M or 0.2 M 2-mercaptoethanol for 3 days when compared to the same enzyme activity in the absence of 2-mercaptoethanol.

Example 4

A reaction mixture containing 2 M glycine and 0.5 mM pyridoxal-5'-phosphate had been degassed by bubbling through $N_2$ gas for 10 minutes before the addition of tetrahydrofolate (2 mM). The pH was maintained at 6.5 by automatic addition of potassium hydroxide (10 N). Redox potential was monitored by redox electrode and maintained by automatic addition of 2-mercaptoethanol (4 N) and a stream of $N_2$ gas over the constantly stirred tetrahydrofolate solution. The level of tetrahydrofolate was followed by HPLC.
Results are shown.

a.  Redox potential:  -270 mV, 37°C

Time (Hours)

|         | 0     | 20   | 48   | 65   |      |
|---------|-------|------|------|------|------|
| (mM) THF | 1.7 g | 1.90 | 1.40 | 1.14 |      |

b.  Redox potential:  -330 mV, 37°C

Time (Hours)

|         | 0    | 7.5  | 22   | 53   | 70   |
|---------|------|------|------|------|------|
| (mM) THF | 1.86 | 2.0  | 2.10 | 2.10 | 2.09 |

c.  Redox potential:  -300 mV, 45°C

Time (Hours)

|         | 0    | 5.5  | 22   | 46   | 53   |
|---------|------|------|------|------|------|
| (mM) THF | 1.96 | 2.02 | 1.72 | 1.6  | 1.4  |

Example 5

The stability of THF and a THF plus formaldehyde mixture were examined under reactor conditions of stirring, nitrogen atmosphere and darkness with the addition of 100mM 2-mercaptoethanol. Table 1 shows that excellent stability was obtained over a period of 20 hours.

Table 1

Stability of THF and Methylene-THF Under Reactor Conditions with the Addition of 100mM 2-Mercaptoethanol

Peak Area at 280nm (arbitrary units)

| Hours | THF Alone THF | THF and Formaldehyde THF | Methylene-THF |
|-------|------|------|------|
| 0 | 24,569 | 8,318 | 20,489 |
| 20 | 27,917 | 6,830 | 20,966 |

## Example 6

One colony of <u>Escherichia coli</u> strain GX 1703 (containing pGx122 with the SHMT gene), which has been& deposited as NRRL No. B-15215 was inoculated into 100ml of culture medium I described below, and was shaken at 37°C overnight.

Culture Medium I:

| | |
|---|---|
| $K_2HPO_4$ | 10.5g/1 |
| $KH_2PO_4$ | 4.5g/1 |
| $(NH_4)_2SO_4$ | 1g/1 |
| Sodium citrate – $2H_2O$ | 0.5g/1 |
| phenylalanine | 20ug/ml |
| Vitamin B$_1$ | 1ug/ml |
| Ampicillin | 100ug/ml |
| $MgSO_4$ | 2mM |
| $FeSO_4$ | 5mg/ml |

0185824

Cells were collected by centrifugation of cell culture medium and used as enzyme source. Glycine (11mM, final concentration) was mixed with tetrahydrofolic acid (5mM, final concentration) pyridoxal-phosphate (1mM), 2-mercaptoethanol (0.2M, final concentration), and formaldehyde (10mM, final concentration) under a nitrogen blanket. The pH was maintained at 7.6 with 0.1M potassium phosphate buffer and the final volume of this reaction mixture was 100 ml. Reaction was started by mixing the above mentioned reaction solution and cells at 37°C with shaking. The redox potential was monitored with a redox probe, and maintained at approximately -300 mV by titration with 2-mercaptoethanol. After 8 hours, 5mM serine was produced (the yield was 45% based on glycine). All of the enzyme activity was retained, and 95% of the THF remained active. Glycine and serine concentrations were determined using high performance liquid chromatography.

### Example 7

One colony of Klebsiella aerogenes strain GX1704 (containing pGx139 with the SHMT gene), which has been deposited as ATCC No. 39214, was inoculated into 100ml of culture medium II described below and was shaken at 30°C overnight.

Culture Medium II.

| | |
|---|---|
| Tryptone | 10g/1 |
| NaCl | 10g/1 |
| Yeast Extract | 5g/1 |
| Glucose | 10g/1 |

Cells were collected by centrifugation of cell culture medium and used as the enzyme source.

The procedure of Example 5 for serine production was followed with the exception that Klebsiella aerogenes was used in place of E. coli. After 8 hours, 7mM serine was produced (the yield was 64% based on glycine). All the enzyme activity was retained, and 92% of the THF remained active.

### Example 8

The procedure of Example 9 was followed with the exception that partially purified serine hydroxylmethyl-transferase from E. coli (strain GX1703 was used. Cells were broken by sonification at 4°C. The supernatant collected after centrifugation was mixed with ammonium sulfate (50% saturation) at 4°C and pH 7.5. After removing the solid by the centrifugation, the enzyme was forced out of the solution by increasing the ammonium sulfate content to 100% saturation. The enzyme was collected by centrifugation and dialyzed. 10mM serine was produced after 4 hours of reaction. The yield was 90% based on glycine. All enzymatic activity was retained, and 94% of the THF remained active.

## Example 9

Whole cells of K. aerogenes were prepared as described in Example 7. HCHO (100mM) was incubated with a reaction mixture containing M glycine 1mM pyridoxal phosphate and K. aerogenes (wet weight 100 mg/ml) at pH 6.5 and 37°C. After 24 hours, only 20% of activity was retained. (Based on β-phenylserine assay see Example 4). On the other hand, the same reaction mixture containing 1mM THF was mixed with 0.1M mercaptoethanol initially and the redox potential of the mixture was controlled at -300mV as described in Example 6, a total amount of 2.1M HCHO was introduced in the period of 47 hours.

Serine, accumulated as high as 210 g/ℓ, was formed and 60% of SHMT activity was recovered.

What is claimed is:

1.  A method of stabilizing tetrahydrofolic acid and serine hydroxymethyltransferase in a reaction mixture for the enzymatic synthesis of L-serine from glycine and formaldehyde which comprises adding to said reaction mixture a stabilizing amount of a reducing agent or a mixture of reducing agents.

2.  A method as claimed in claim 1, wherein the reducing agent is selected from the group consisting of ascorbic acid, thioglycolate, 2-mercaptoethanol, 1,3-dimercaptopropan-2-ol, 2,3-dimercaptopropanol, 1,2-dimercaptoethane, dithiothreitol, dithioerythritol, mercaptoethanol, L-thiazolidine-4-carboxylic acid and aminoethylisothiouronium bromide.

3.  A method as claimed in claim 1, wherein the reducing agent is 2-mercaptoethanol.

4.  A method as claimed in claim 1, 2 or 3, wherein the concentration of reducing agent which is added to the reaction mixture is from 0.01M to 0.2M.

5.  A method as claimed in claim 1, 2 or 3, wherein the concentration of reducing agent which is added to the reaction mixture is from 0.02M to 0.1M.

6.  A method as claimed in claim 1, 2 or 3, wherein the amount of reducing agent added is sufficient to maintain the redox potential of the reactants between -300mV and -500mV.

7.  A method as claimed in claim 1, 2 or 3, wherein the amount of reducing agent added is an amount sufficient to maintain the redox potential of the reactants at between -300mV and -350mV.

8.  A method as claimed in claim 1, 2 or 3, wherein the reactants are prevented from contacting air during the reaction.

9.    A method as claimed in any of the preceding claims wherein tetrahydrofolic acid is provided to the reaction medium at a concentration in excess of the amount necessary to catalyze the reaction and formaldehyde is fed to the reaction medium at a rate equal to or less than the rate at which it is consumed in the reaction.

10.    In a process for preparing serine wherein formaldehyde, tetrahydrofolic acid and glycine are reacted in the presence of serine hydroxymethyltransferase, the improvement which comprises providing THF to the reaction medium at a concentration in excess of the amount necessary to catalyze the reaction and feeding formaldehyde to the reaction medium at a rate equal to or less that the rate at which it is consumed in the reaction.

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | BIOLOGICAL ABSTRACTS, vol. 67, no. 9, 1979, page 5556, no. 55725, Philadelphia, US; R.J. POLLOCK et al.: "Dihydropteridine reductase may function in tetrahydrofolate metabolism" & J. NEUROCHEM. 31(1): 115-124, 1978 * Abstract * | 1-3 | C 12 P 13/06 C 12 N 9/96 |
| | --- | | |
| A | GB-A-2 130 216 (GENEX CORP.) * Claims; page 3, lines 6-15 * | 1,9,10 | |
| | --- | | |
| A | FR-A-2 078 741 (KYOWA HAKKO KOGYO K.K.) * Claims; page 3, lines 4-13 * | 1 | |
| | ----- | | |

TECHNICAL FIELDS SEARCHED (Int. Cl.4)

C 12 P
C 12 N

The present search report has been drawn up for all claims

| Place of search | Date of completion of the search | Examiner |
|---|---|---|
| THE HAGUE | 03-09-1985 | DELANGHE L.L.M. |